# EUROPEAN PATENT SPECIFICATION

(11) **EP 1 509 255 B1**
(45) Date of publication and mention of the grant of the patent: **16.04.2008**
(21) Application number: 03738247.0
(22) Date of filing: 02.06.2003
(51) Int. Cl.: A61K 51/10, A61K 47/48

(54) **METHODS AND COMPOSITIONS FOR INTRAVESICAL THERAPY OF BLADDER CANCER**
METHODEN UND MEDIZINISCHE ZUSAMMENSETZUNGEN ZUR INTRAVESIKALEN BEHANDLUNG VON BLASENKREBS
METHODES ET COMPOSITIONS DE TRAITEMENT INTRAVESICAL DU CANCER DE LA VESSIE

(30) Priority: 03.06.2002 US 384391 P
(43) Date of publication of application: 02.03.2005
(73) Proprietor: Immunomedics, Inc., Morris Plains, NJ 07950 (US)
(72) Inventor: GRIFFITHS, Gary, L., Morristown, NJ 07690 (US); GOLDENBERG, David, M., Mendham, NJ 07945 (US)
(74) Representative: Bohmann, Armin K.
(86) International application number: PCT/GB2003/002387
(87) International publication number: WO 2003/101496

(56) References cited:
- WO-A-02/40507
- WO-A-99/66951
- WO-A-02/082041
- WO-A-03/011342
- WO-A-03/057829
- WO-A-03/059397
- US-A- 4 444 744
- MANETTI ET AL: "Intracellular uptake and catabolism of anti-IgM antibodies and bi - specific antibody -targeted hapten by B-lymphoma cells" INTERNATIONAL JOURNAL OF CANCER, NEW YORK, NY, US, vol. 63, no. 2, 9 October 1995 (1995-10-09), pages 250-256, XP002120770 ISSN: 0020-7136
- M HILLAIRET DE BOISFERON ET AL.: "Pretargeted radioimmunotherapy using 131I-lablelled bivalent hapten-bearing peptides" LETTERS IN PEPTIDE SCIENCE, vol. 4, 1997, pages 331-339, XP008021448
- KARACAY H ET AL: "Experimental pretargeting studies of cancer with a humanized anti-CEA * Murine anti-(In-DTPA) Bispecific antibody construct and a 99mTc/188Re-labeled Peptide" BIOCONJUGATE CHEMISTRY, AMERICAN CHEMICAL SOCIETY, WASHINGTON, US, vol. 11, 2000, pages 842-854, XP002230983 ISSN: 1043-1802
- JANEVIK-IVANOVSKA E ET AL: "BIVALENT HAPTEN-BEARING PEPTIDES DESIGNED FOR IODINE-131 PRETARGETED RADIOIMMUNOTHERAPY" BIOCONJUGATE CHEMISTRY, AMERICAN CHEMICAL SOCIETY, WASHINGTON, US, vol. 8, no. 4, 1997, pages 526-533, XP000929131 ISSN: 1043-1802
- HUGHES ODM ET AL.: "Targeting superficial blader canncer by the intravesical adminiatration of Copper-67-labelled anti-MUC1 Mucin monoclonal antibody" JOURNAL OF CLINICAL ONCOLOGY, vol. 18, no. 2, 2000, pages 363-370, XP008021360 cited in the application
- HOSONO M ET AL: "Two-step targeting and dosimetry for smalö cell lung cancer xenograft with ant-NCAM/antihistamine bispecific antibody and radioiodinated bivalent hapten" THE JOURNAL OF NUCLEAR MEDECINE, vol. 40, no. 7, 1999, pages 1216-1221, XP008021447
- GESTIN J F ET AL: "Two-step targeting of xenografted colon carcinoma using a bispecific antibody and 188Re-labeled bivalent hapten: biodistribution and dosimetry studies" JOURNAL OF NUCLEAR MEDICINE, SOCIETY OF NUCLEAR MEDICINE. NEW YORK, US, vol. 42, no. 1, January 2001 (2001-01), pages 146-153, XP002178931 ISSN: 0161-5505
- MURRAY A ET AL.: "Production and characterization of 188-Re-C595 antibody for radioimmunotherapy of transitional cell bladder cancer" THE JOURNAL OF NUCLEAR MEDICINE, vol. 42, no. 5, 2001, pages 726-732, XP001165620 cited in the application
- VARVARIGOU AD ET AL.: "Immunotargeting of urothelial cell carcinoma with inravesically administered Tc-99m labeled HMFG1 monoclonal antibody" CELL. BIOPHYSICS, vol. 24/25, 1994, pages 75-81, XP008021505
- KUNKLER RB ET AL: "Targeting of bladder cancer with monoclonal antibody NCRC48-a possible approach for intravesical therapy" BRITISH JOURNAL OF UROLOGY, vol. 76, 1995, pages 81-86, XP008021496
- BAMIAS A ET AL.: "Intravesical adminiastration of iNDIUM-111-labelled HMFG2 monoclonal antibody in superficial bladder carcinomas" INTERNATIONAL JOURNAL OF CANCER, vol. 54, 1993, pages 899-903, XP008021497
- GRUAZ-GUYON A ET AL: "RADIOLABELED HAPTEN-DERIVATIZED PEPTIDES FOR TUMOR IMAGING WITH BISPECIFIC ANTIBODY CONJUGATES" PEPTIDES, PROCEEDINGS OF THE 21ST EUROPEAN PEPTIDE SYMPOSIUM, LEIDEN, NL, 1991, pages 822-825, XP000925374
- KARACAY H ET AL : "Pretargeting for cancer radioimmunotheray with bispecific antibodies: roles of the bispecific antibody's valency for the tumor target antigen" BIOCONJUAGTE CHEMISTRY, vol. 13, 2002, pages 1054-1070, XP001157898
- SHARKEY RM ET AL.: "A universal pretargeting system for cancer detection an d therapy using bispecific antibody" CANCER RESEARCH, vol. 63, 2003, pages 354-363, XP001164252
- IMMUNOMEDICS: "clinical overview" IMMUNOMEDICS, [Online] XP002257617 Retrieved from the Internet: <URL:www.immunomedics.com> [retrieved on 2003-09-19]

## Description

### Background

Bladder cancer is a relatively common cancer, particularly prevalent among men, and its incidence is lowly increasing. Superficial cancers are generally treated by endoscopic resection, although virtually all patients develop new tumors in the bladder, many of which progress to a higher stage. Further treatments over time can include further resections, radiation, and various intravesical therapies including those using chemotherapy agents and bacillus Calmette-Guerin. All therapies have adverse side effects. Ultimately, disease can spread such that a cystectomy (removal of the entire bladder and multiple surrounding tissues) is necessitated. Because bladder cancer is often diagnosed at an early stage it is amenable to, and often responsive to, certain treatments administered intravesically. Unfortunately, none is curative, and few in fact provide regressions of any meaningful duration. Further, when the bladder carcinoma spreads beyond this organ, virtually all patients succumb to this metastatic disease. Even when the bladder carcinoma remains within the bladder but penetrates beyond the superficial epithelium into the deeper muscular layer, potential for cure relies only on total bladder extirpation, which then requires a urinary pouch to be made from the patient's gut, and which provides much difficulty to the patient and a major effect on the patient's quality of life.

Radioimmunotherapy (RAIT) with monoclonal antibodies (mAbs) is a very promising modality for the targeted and specific treatment of various, cancers, ant promise substantially improved outcomes compared to standard radiotherapy and chemotherapy approaches to cancer treatment. It does, however, suffer from the disadvantage that when a radiolabeled mAb is injected into a cancer patient a finite amount of time is needed for the radioimmunoconjugate to both maximize in tumor target tissue, and clear from background tissues and circulation. During this time, which is quite long for an intact radiolabeled immunoglobulin IgG and somewhat shorter for radiolabeled IgG fragments and sub-Fab' fragments, the patient is exposed to non-disease targeted radiation. This non-targeted radiation, primarily received during the mAb localization phase, translates directly to radiotoxicity. This, in turn, limits the total amount of radiolabeled mAb that can be administered, preventing dose escalation to achieve optimal RAIT, which can require tumor doses in the range of 50 to 80 Gy, because most solid tumors (carcinomas) are relatively radioresistant, as compared to hematopoietic neoplasms.

To overcome this problem, delivery of radionuclide has been separated from the initial targeting step in a method generally called pretargeting. In this system a localization moiety, typically a multispecific antibody (msAb) that has at least one arm that binds to a tumor antigen and at least one other arm that binds to a low molecular weight hapten (example: a bispecific antibody (bsAb)), is given to a patient, and allowed to maximize in tumor tissue while also clearing normal tissues. Some time later the low molecular weight hapten is given in a radiolabeled form. The latter localizes to the multispecific antibody pretargeted to the tumor but otherwise species to the tumor target via the multispecific antibody almost immediately post-administration while the unbound radioactivity is eliminated, via the kidneys and urine, dramatically shifts the therapeutic ratio in a positive manner. Increased amounts of radioactivity can be directed to the tumor target, while normal tissues are spared and overall toxicity thereby decreased.

Intravesical RAIT has been proposed and investigated previously for the treatment of bladder cancer. See Murray et al., J Nucl Med 2001;42:726-732. 2001; Hughes, et al., J. Clin. Oncol., 18:363-370, 2000, and Syrigos, et al., Acta Oncol., 38:379-382, 1999. As with conventional RAIT, a conjugate of radionuclide and monoclonal antibody is used, being delivered via the urethra directly into the bladder. A significant reduction in toxicity is to be expected since there is no exposure of other major internal organs such as bone marrow, liver, spleen and lungs, to the radioactive immunoconjugate. The use of a direct conjugate of a radionuclide and a monoclonal antibody for the bladder cancer indication therefor offers a significant potential advantage over standard RAIT directed to most other cancers. However, in a prior attempt at this approach, see above, high tumor uptake of the radiolabeled antibody was only achieved for a short time, and dissipated by 24 h (Hughes 2000). In Murray 2001, moreover, the radioimmunoconjugate used was found to be unstable, and no evidence of antitumor activity was reported. Thus, although localization of radioactivity to bladder cancer could be achieved by intravesical administration, no evidence of antitumor activity has been achieved to date, and any targeting observed has been limited to superficial bladder cancer and for a period of timed would be insufficient for any successful therapy with the radiation emitted from the radionuclide.

WO99/66951 discloses a carrier molecule IMP192 and example 17 concerns a bispecific anti-CEA x anti-di DTPA antibody. Journal of Nuclear Medicine (2001), 42(5), 726-732 describes the use of bispecific antibodies on a human colon carcinoma cell line; Journal of Clinical Oncology (2000), 18(2), 363-370; Cell Biophysics (1994), 24/25, 75-81; British Journal of Urology (1995), 76, 81-86; International Journal of Cancer (1993); 54, 899-903 describe the treatment of superficial bladder cancer with labelled antibodies administered intravesically.

### Summary of the Invention

One aspect of the invention is the use of a therapeutically effective amount of a bispecific antibody comprising at least one targeting arm that binds a bladder cancer antigen and at least one capture arm that binds a carrier conjugated to one or more therapeutic agents in the preparation of a medicament for the treatment of bladder cancer in a patient; wherein the bispecific antibody is administered via the urethra and is optionally allowed to localize at the site of the bladder cancer, and optionally any free bispecific antibody is allowed to substantially clear from the patient.

Another aspect of this invention is the use according to claim 28.

### Description of the Invention

A major problem that exists with all RAIT protocols, that still remains with the above-mentioned intravesical RAIT, and to which this invention is directed, remains unaddressed. This problem is now addressed as described in detail below by the novel combination of multispecific antibody technology, the approach of intravesical administration of targeting and therapy reagents, the optional systemic delivery of a second therapeutic carrier, and judicious choice of carriers for useful RAIT nuclides. The therapeutic agents delivered by the current invention include, but are not limited to, radionuclides.

The major problem is that absolute tumor uptake of mAbs as a percentage of the dose given are usually very low in a clinical setting, often 0.01 to 0.00001 % injected dose per gram of tissue, and that the residence time of the radioactivity in the tumor is often not sufficient to achieve the radiation doses needed. Thus, a very small portion of the radiolabeled msAb that is injected is actually localized to target tissue for a relatively short time, while a very large excess distributes throughout normal tissues, and causes toxicity. Localization is the process by which antibodies are hallowed bind to their target tissue and generally occurs within 1 to 10 hours. By adoption of intravesical RAIT one can avoid systemic toxicity, while obtaining similar tumor uptake values, and therefore shift the therapeutic ratio in the desired direction. However, that absolute tumor uptake remains very low, and is finitely limited to the number of antigen sites that can be targeted by the targeting antibody. Also, it has been found (Hughes 2000) that the duration of exposure of the tumor to the radioactivity delivered by intravesical RAIT is less than 24 hours, thus being insufficient for effective radiation of the cancer. As described above (Murray 2001), others attempting this approach of intravesical RAIT for bladder cancer therapy have not been able to use stable radioconjugates, thus failing to deliver adequate and specific radiation to the tumor. Thus, other methods are needed to solve these problems. In addition to these problems, there is also a deficiency in that not every antibody molecule is associated with a radionuclide molecule. This means that a mAb molecule that is not carrying a radioactive payload targets most of the antigenic sites that are available. Without internalization and/or recycling, if one in ten mAb molecules carry a radionuclide atom, then only one in ten antigen sites can be targeted with a radionuclide. One-in-ten mAb molecules bearing a radionuclide is in fact a very good mAb-to-radionuclide ratio in practical terms, since the ratio often can be one-in-one hundred or even lower. For instance, when one considers a sample of mAb labeled with the therapeutic radionuclide rhenium-188 at 1 mCi per mg of protein, about one in two hundred mAb molecules is actually associated with a radioactive Re-188 atom. Clearly, there would be an improvement in intravesical RAIT if more radionuclide could be directed to the antigen sites where it is needed, without unwanted blockade of the limited numbers of antigen sites on those tumors, and to achieve a longer duration of exposure of the bladder cancer cells to the radiotherapeutic.

By using pretargeting, one eliminates the need for a targeting mAb to carry the radioactive payload. Since mAbs are delicate biological molecules that are readily impaired in their ability to bind to their antigenic targets if over-loaded or subjected to harsh conditions related to chemistry or radiolytic events, the use of multispecific antibodies (msAbs) offers a unique chance to overcome the practical problem of delivery rapacity that is evident with intravesical RAIT using direct conjugates. Conjugates are formed when a recognition hapten binds to a multispecific antibody.

The use of msAbs as the targeting vectors, in separating the mAb targeting step from the radionuclide-targeting step, allows greatly expanded freedom in designing radionuclide-binding moieties. Those embodiments are described in detail below. In addition, the particularly preferred embodiment wherein the radionuclide-binding moiety is deposited directly into the bladder, via the urethra, rather than through the blood system removes several constraints that exist with respect to radionuclide complex stability in blood and tissue, systemic pharmacokinetics, and any unwanted metabolism in non-targeted tissues. A complex is formed when a radionuclide binds to a chelate. Moreover, the administration of a radionuclide-bearing moiety after the msAb has localized to the tumor results in a longer duration of radiation of the tumors, including deeper-seated tumors if the appropriate radionuclide and path-length of radiation emitted is selected. Optionally, if seeding of tumor outside of the bladder is suspected, or if a prevention of such spread is desired, then the second radionuclide-binding moiety can be given systemically also.

A superior RAIT can be achieved using the following method: msAbs are preferably administered through the urethra of bladder cancer patients, allowed to localize and maximize to tumor tissue over a short period. After evaluation of unbound msAb, a radiolabeled moiety is given, either intravenously and/or intravesically, and allowed a short period to bind to pretargeted msAb. Excess radiolabeled moiety is excreted, leaving only tumor-bound radioactivity to decay. This process can be repeated, so as to increase the dose of radiation delivered to tumor. In an alternative embodiment, msAbs are premixed with the radiolabeled recognition moiety and injected intravesically. After excretion of unbound msAb, the remaining radioactivity decays at the site of tumor deposits. These approaches will deliver ionizing radiation selectively to the cancer cells for periods exceeding 24 hours, and in some cases, exceeding 48 hour. This is in part because the radioimmunoconjugates used are sufficiently stable to deliver more radioactivity to tumor than to other normal tissues.

In addition, any aspect of this invention can further comprise the following. Determining the amount of multispecific antibody localized into the bladder prior to administering said carrier conjugated to one or more therapeutic agents. Also any method of the present invention can be performed wherein the amount of multispecific antibody localized into the bladder is determined by quantifying the amount of multispecific antibody recovered from excretion. Any method of the present invention can be performed wherein the amount of multispecific antibody localized into the bladder is determined by imaging the patient and wherein the multispecific antibody further comprises a tracer nuclide. Tracer nuclides can be selected from F-18, Ga-67, Ga-68, Tc-99m, In-111, I-123, I-131, or gadolinium.

### Specific Targeting

The presence of accessible tumor sites in bladder cancer that can be specifically targeted without passage of the targeting agent through the central circulatory and catabolic systems of the body means that a substantial amount, and in some cases almost all, of the msAb administered into a patient's bladder can be localized to tumor tissue. Therefore, the low specific target uptake/high non-target distribution (0.01-0.0001% ID/g in specific target tissue versus the remainder of an injected dose in non=target.tissue) seen with any systemic msAb approach is rendered irrelevant. Empiric testing of a patient, using a variety of standard methods, can be used to determine the extent of disease localized in the bladder and an appropriate amount of targeting antibody then given. Determining the amount of antibody localized into the bladder using methods know in the art, such as by biopsy or imaging can be used. If this were standard systemic RAIT the patient would then receive a nuclide-msAb conjugate wherein one in every 10-1000 mAb molecule would actually carry a radionuclide atom capable of destructive decay.

In the current invention the above targeting step is performed with a msAb that has one arm reactive against a tumor antigen expressed on the bladder cancer. Once excess msAb has been substantially cleared, and a high number of available antigen tumor sites have been saturated by the administered msAb, the radiolabeled hapten recognized by the msAb is given. Antibodies are considered substantially cleared when approximately 90% or more of the administered antibody has left the body of the patient. The dose of the radiolabeled hapten can be determined from the amount of msAb previously localized into the bladder, In turn, the latter can be readily determined from the dose of msAb administered and the dose recovered during the excretion phase, precedent to radiolabeled hapten administration. In one preferred embodiment, the dose of the msAb retained in the bladder can be determined using a msAb radiolabeled with a small amount of tracer radionuclide, with the patient optionally imaged prior to administration of the radiolabeled hapten. It must be appreciated that the act of decoupling the radionuclide from the disease targeting mAb also uncouples the constraints placed on targeting by maximum achievable specific activity of direct mAb radiolabeling. In other words, if the radiolabeled hapten can be prepared at a 1:1 nuclide-to-recognition hapten ratio, each msAb on the tumor tissue can then localize one radionuclide atom. By both using the premixing and pretargeting methods of this invention, approximately equimolar ratios of antibody and active agent can be delivered. An approximate equimolar ratio can be from about 1:1 to about 1:10 and all ratios, such as 1:2, 1:3, etc., that are between 1:10. Where the molar ratios are below 1:10, they are more preferably below 1:6 and more preferably below 1:3. Furthermore, if more than one radionuclide atom can be associated with each recognition hapten, the amount of radionuclide localized per msAb localized can even exceed this 1:1 ratio. The latter can be readily achieved by multiply substituting radionuclides onto a moiety that has only one or two recognition units.

The msAb preferably has an adequate affinity for both antigen tumor tissue and for the radiolabeled recognition hapten. Generally, each targeting specificity should be able to bind to its recognition moiety over an expended period, which implies a Kₐ generally at or above 10⁻⁷ M. However, with the current indication slightly lower Kₐs are also useful, and may even be preferable under certain circumstances, such as when deeper penetration of tissue is requited, since it is well known that a targeting Ab with a greater affinity tends to bind less well to tissue. Also, in this regard, it must be appreciated that msAb fragments and sub-fragments are also especially useful in the practice of the current invention since they inherently have greater tissue penetration properties than larger molecules such as those the size of IgGs. In standard systemically administered RAIT and msAb RAIT, it is well known that administration of smaller sized targeting vectors leads inevitably to faster blood clearance characteristics and lower target tissue uptakes, further reducing absolute target uptake from the already low absolute levels achievable with a radiolabeled IgG or a msAb based on IgG x IgG. Since the msAbs of the current invention are given intravesically, blood clearance characteristics are irrelevant, and fragments and sub-fragments are rendered more useful.

### Multispecific Antibodies

MsAbs can include antibody fragments, subfragments and combinations thereof The antibody fragments are antigen binding portion of an antibody, such as F(ab')₂, F(ab)₂, Fab', Fab. The antibody fragments bind to the same antigen that is recognized by the intact antibody. For example, an anti-CD22 monoclonal antibody fragment binds to an epitope of CD22. The msAbs of the present invention also include, but are not limited to, IgG x IgG, IgG x F(ab')₂, IgG x Fab', IgG x scFv, IgG sFv, F(ab')₂ x F(ab')₂. Fab' x F(ab')₂. Fab' x Fab', Fab' x scFv, Fab' x sFv, (sFv x sFv)₂, sFv x sFv, and scFv x scFv bi-specinc monoclonal antibodies (bismAbs). Also, species such as scFv x IgG x scFv and Fab' x IgG x Fab', scFv x F(ab')₂ x scFv and Fab' x F(ab')₂ x Fab' are included Most preferably, site-specific attachment sites on the IgG or F(ab')₂ of one or both monoclonal antibodies (mAbs) can be utilized, such as an engineered carbohydrate or an engineered or liberated free thiol group. Since these mAbs are dimeric they can be coupled with two moles of the second mAb. For instance, a mAb directed towards carcinoembryonic antigen (CEA), anti-CEA F(ab')₂, having an engineered light-chain carbohydrate can be oxidized and converted using a hydrazide-maleimide cross-linker to a derivatized anti-CEA F(ab')₂ having at least one pendant maleimide group per each light chain. This specie is coupled to an anti-chelate Fab'-SH at a 1:2 molar ratio, at least, such that an anti-chelate-Fab' x anti-CEA-F(ab')₂-anti-chelate Fab' conjugate is produced. The resultant msAb is bivalent with respect to the target tissue and the polymer conjugate. At their smallest, msAbs constructed with peptide molecular recognition units directed against each specificity, including also diabodies, triabodies, tetrabodies, quintabodies. It is further understood that the use of the term "msAb" in the present disclosure encompasses multi-specific antibodies and multi-specific antibody fragments.

The term "antibody fragment" also includes any synthetic or genetically engineered protein that acts like an antibody by binding to a specific antigen to form a complex. For example, antibody fragments include isolated fragments, "Fv" fragments, consisting of the variable regions of the heavy and light chains, recombinant single chain polypeptide molecules in which light and heavy chain variable regions are connected by a peptide linker ("sFv proteins"), and minimal recognition units consisting of the amino acid residues or related peptides that mimic the hypervariable region.

The msAbs of the current invention may be monoclonal or polyclonal in nature, but preferably monoclonal. Furthermore, the targeting arm and the capture arm of the msAb may be monoclonal or polyclonal in nature. Preferably, either the target arm or the capture arm is monoclonal. Most preferably, the target arm and the capture arm are both monoclonal.

The msAb of the current invention may be engineered to possess a label. Examples of labels that the msAb may possess include, but are not limited to, a labeling ligand such as the biotin-streptavidin complex and radioisotopes. Advantageously, the msAb of the current invention is radiolabeled to facilitate tracking of localization and clearance.

In any aspect of the present invention, the multispecific antibody can comprise one or more antibody fragments or sub-fragments. The multispecific antibody can be selected from the group consisting of IgG x Fab', IgG x sFv, F(ab')₂ x Fab', Fab' x Fab', Fab' x sFv, (sFv x sFv)₂, sFv x sFv, diabody, triabody, tetrabody, and quintabody. Also the multi-specific antibody can have more than one targeting arm. The more than one targeting arm can be F(ab')₂ x Fab'.

MsAbs useful in the current invention are also understood to encompass msAbs with more than one targeting arm such as a F(ab')₂ x Fab' fragment. Thus, one arm can be targeted against the recognition hapten with two arms directed toward a tumor-associated antigen, or *vice versa*. In addition, the F(ab')₂ part of the F(ab')₂ x Fab' fragments (assuming the Fab' part is directed against the radiolabeled hapten) can be directed against two distinct epitopes on the same antigen (e.g, CEA) or two distinct antigens (e.g., CEA and MUC1). It, itself can thus be multispecific in terms of targeting ability, with one Fab' or sFv arm directed against one tumor antigen and one directed against a second tumor antigen on target tissue. In addition, one targeting arm of this F(ab')₂ or (sPv x sFv)₂ sub-species can be directed against a tumor antigen while the second targeting arm is directed against a separate type of antigen, such as a vascular antigens epitope, present on bladder tumors.

Also useful for this invention are the bispecific fusion proteins described in U.S. Application Nos. 09/911,610, filed July 25, 2001, 09/337,756, filed June 22, 1995 and 09/823,746, filed April, 3, 2001. Other antibodies and useful compositions and method for the present invention include a mutant bispecific antibodies, containing an IgG component and two scFV components, wherein the Fc-hinge fragment of the IgG contains one or more amino acid mutations in the CH2-CH3 domain interface region, the mutant fusion bsAb, hMN14IgG^{(I253A)}-(734scFv)₂ and the subject matter disclosed in U.S. Provisional Application 60/361,037, filed March 1, 2002.

### Target Antigens

Target antigens useful under the current invention encompass any type of epitope that is present to a greater extent on bladder tumor tissue than on normal bladder tissue, or present to a greater extent on vascular tissue within a bladder tumor compared to normal bladder tissue. Exemplary epithelial antigens are carcinoembryonic antigen (CEA), CD44, MUC-1, epithelial glycoprotein (EGP), epidermal growth factor receptor (EGFR), vascular endothelial growth factor receptor (VEGFR), human milk fat globulin antigens (HMPG1 and HMFG2), and tumor necrosis substances (e.g., histones). Also, antigens particularly associated with bladder cancer include MUC-2, MUC-3, MUC-4; Le-y, TAG-72, IL-6, and VEGF. In addition to these receptors (or ligands), the corresponding ligand (or receptor), or ligand-recepthr complex can serve as useful targets for antibodies. For example, in addition to the VEGF receptor, VEGF or the VEGFR:VEGF complex can be useful targets for antibodies. Antibodies to many of these antigens have been described in the scientific literature (Goldenberg, J Nucl Med 2002;43:693-713). Additional antibodies include products of oncogenes, and antibodies against tumor necrosis substances, such as described in patents by Epstein et al. (U.S. Pat. Nos. 6,071,491, 6,017,514, 5,019,368 and 5,882,626). Also of use are antibodies against markers or products of oncogenes, or antibodies against angiogenesis factors, such as VEGF. VEGF antibodies are described in Thorpe et al, U.S. Pat. Nos. 6,342,221, 5,965,132 and 6,004,554, and are incorporated by reference in their entirety. In any aspect of the present invention the bladder cancer antigen can be selected from the group consisting of carcinoembryonic antigen (CEA), CD44, MUC-1, epithelial glycoprotein (EGP), epidermal growth factor receptor (EGFR), vascular endothelial growth factor receptor (VEGFR), tumor necrosis substances, and human milk fat globulin antigens (HMFG1 and HMFG2).

### Therapeutic Agents

In any aspect of the present invention, therapeutic agents can include radionuclides. Exemplary radionuclides include Sc-47, Ga-67, Y-90, Ag-111, In-111, Sm-153, Tb-166, Lu-177, Bi-213, Ac-225, Cu-64, Cu-67, Pd-109, Ag-111, Re-186, Re-188, Pt-197, Bi-212, Bi-213, Pb-212 or Ra-223.

Other therapeutic agents can include toxins or chemotherapeutic agents, especially those that are useful in treating cancer. The toxin may include ricin, abrin, ribonuclease, DNase I, Staphytococcal enterotoxin-A, pokeweed antiviral protean, gelonin, diphtherin toxin, Pseudomonas exotoxin, or Pseudomonas endotoxin.

Chemotherapeutic agents, for the purpose of this disclosure, include all known chemotherapeutic agents. Known chemotherapeutic agents include, at least, the taxanes, nitrogen mustards, ethylenimine derivatives, alkyl sulfonates, nitrosoureas, triazenes; folic acid analogs, pyrimidine analogs, purine analogs, vinca alkaloids, antibiotics, enzymes, platinum coordination complexes, substituted urea, methyl hydrazine derivatives, adrenocortical suppressants, or antagonists. More specifically, the chemotherapeutic agents may be steroids, progestins, estrogens, antiestrogens, or androgens. Even more specifically, the chemotherapy agents may be azaribine, bleomycin, bryostatin-1, busulfan, carmustine, chlorambucil, cisplatin, CPT-11, cyclophosphamide, cytarabine, dacarbazine, dactinomycin, daunorubicin, dexamethasone, diethylstilbestrol, doxorubicin, ethinyl estradiol, etoposide, fluorouracil, fluoxymesterone, gemcitabine, hydroxyprogesterone caproate, hydroxyurea, L-asparaginase, leucovorin, lomustine, mechlorethamine, medroprogesterone acetate, megestrol acetate, melphalan, mercaptopurine, methotrexate, methotrexate, mithramycin, mitomycin, mitotane, phenyl butyrate, prednisone, procarbazine, semustine streptozocin, tamoxifen, taxanes, taxol, testosterone propionate, thalidomide, thioguanine, thiotepa, uracil mustard, vinblastine, or vincristine, and BCG.

Chemotherapeutic agents can be conjugated to one or more haptens using standard chemical modifications, selected based on the structure of the individual drug, together with the structure of the peptide or polymer to which it is to be attached. Such standard methodologies can be readily obtained from standard books on organic syntheses (e.g. see in R.C. Larock, Comprehensive Organic Transformations, VCH Publishers, NY, 1989, or J. March, Advanced Organic Chemistry, Wiley-Interscience, NY, 1985), which are easily obtainable by those skilled in the art. To cite an example, the structure of the standard chemotherapy drug doxorubicin can be illustrative. For instance, the anthracycline analog doxorubicin has a free keto- group in its 13-position, a free amino group on its glycan ring and an alkyl hydroxyl group in the side-chain C-14. Any of these could be used to couple doxorubicin to the backbone of a hapten-bearing moiety. More specifically, the free amino group on the glycan might be coupled, forming an amide, to a carboxyl-moiety on the hapten, for instance to a carboxyl-containing polymer containing multiple aspartyl or glutamyl residues. The ketone might be coupled to a hapten-peptide that also contains a free hydrazinyl-moiety, forming a hydrazone bond, for instance to a tetrapeptide that has an N-terminal hydrazine. The hydroxyl group might be coupled to a carboxyl-containing hapten-peptide, forming an ester bond, for instance to a short peptide that has a glutamyl or aspartyl residue. In addition, any of these groups on the doxorubicin can be activated using standard cross-linking agents, such as those obtainable from *Pierce Chmical Company* (Chicago, IL). For example, a heterobifunctional cross-linking agent that comprises a hydrazine and a maleimide can be reacted with the 13-keto group of the doxorubicin to form an intermediate doxorubicin-linker adduct (hydrazone-linked), that bears a maleimide group. As is well known, maleimide groups react with free thiol groups under facile conditions at neutral pH, so the doxorubicin-linker-maleimide adduct can then be reacted with thiol-containing haptens, hapten, peptides or hapten-polymers to generate suitable conjugates. This, and similar strategies for linking drugs and targeting agents are well-known in the art (e.g Willner et al. Bioconjug. Chem., 4:521-527, 1993).

### Antibody Preparation

Antibodies to secondary recognition haptens can be prepared using standard methods of immunologic priming followed by generation of hybridoma clones producing monoclonal antibodies of interest. In this manner, various specific antibodies have been made and produced in bulk, and these include antibodies to the metal-chelate complexes indium-diethylenetriaminepentaacetic acid (In-DTPA), and yttrium-1,4,7,10-tetraazacyclododecane-N,N'N",N"'-tetraacetic acid, (Y-DOTA), and to other diverse species such as histamine-succinyl-glycine (HSG), biotin and fluorescein. The current invention includes in its scope any antibody to and secondary recognition hapten, including multispecific antibodies that can bind to any epitope on a large structure, such as a polymer. Specificities and affinities of the tumor targeting and the secondary recognition mAb can be pre-selected using standard methods of phage display, and human msAbs of desired properties obtained thereby. Specific antibodies can be affinity matured by techniques known in the art in order to enhance affinity and on- and off-rates.

MsAbs of the current invention are prepared by well-known methods using chemical linkages, somatic methods, or by molecular biology derived expression systems, producing proteins in appropriate host organisms. It is to be appreciated that the source or the mode of production of the msAb is not central to the current invention. Thus the term msAb is herein intended to encompass any multivalent, multispecific, targeting antibody or fragment/subfragment, and specifically includes divalent x divalent and trivalent x monovalent and trivalent x divalent species, multispecific mini-antibodies, diabodies, triabodies, tetrabodies, quintabodies, and scFv x scFv tandems.

In a preferred embodiment, the targeting msAb can be radiolabeled for easier quantitation of the amount taken up in the tumor tissue. This can be done by simple subtraction or it may be done using a well-known imaging technique, in either case, after elimination of the unbound radiolabeled msAb. Using a penetrating radionuclide, computed tomographic (CT) or single photo emission computed tomographic (SPECT), or positron emission tomographic (PET) imaging can be performed prior to administration of the radionuclide recognition hapten conjugate. In any event the purpose of this quantitation is to better gauge the amount of radiolabeled recognition hapten that is appropriate for a particular patient. Radionuclides useful for imaging under this embodiment include, but are not restricted to, F-18, Ga-67, Ga-68, Tc-99m, In-111, I-123 or I-131.

Recognition haptens of the current invention only need to have at least one epitope that is recognized by at least one arm of the pretargeted msAb. This is quite different from standard msAb RAIT protocols, wherein bivalent hapten binding is very important. In the intravesical approach there is substantially less competitive breakdown of msAb-recognition hapten complex, due to the absence of numerous serum components in bladder contents. In addition, metabolic clearance processes can be discounted in the case of bladder cancer. When msAb RAIT therapy is performed systemically it has been shown that the recognition needs to be bivalent in nature. If it is monovalent, it does not bind well enough to pretargeted msAb to be retained for a long time in the tumor target. If it is tri- or higher valent then the risk is that formation of high molecular weight complexes in the serum will lead to premature clearance of the radiolabeled recognition hapten, primarily into the liver and spleen of the patient, resulting in poor tumor uptake and non-specific radiotoxicity. The current invention therefore encompasses recognition haptens of any valency to msAb from one upward, with minimal or negligible concern for the dual problems of poor retention and premature clearance.

Because of the issues just discussed, considerably more freedom can be applied to the design of recognition haptens for use in msAb-pretargeted RAIT. In the simplest form, a conjugate of the recognition hapten and the radionuclide can now be used since monovalent binding is useful within the scope of the invention. Examples of this are msAbs bearing an arm reactive with metal ion chelates with DTPA or DOTA, anti-biotin mAbs for use with biotin-chelate conjugates, and anti-HSG mAbs for use with HSG-chelate conjugates. In these examples the metal is radioactive and bound strongly by the chelating agent. It is known that metal complexes of low molecular weight chelators can be prepared at near 1:1 ratios of metal to chelator, if the metal is purified appropriately and the chelator is chosen appropriately. Radiometals useful in the current invention include those that decay with particulate emission such as alpha and beta emitters, and/or with low energy gamma ray emission (Auger emitters). They include the following, in a non-exhaustive list: Sc-47, Ga-67, Y-90, Ag-111, In-111, Sm-153, Tb-166, Ln-177, Bi-213 and Ac-225. For radiolabeling, it should also be borne in mind that any of these metals can be initially complexed by an excess of a chelating agent, with the excess chelating agent then removed from the metal chelate. The separation is usually based on an ion-exchange procedure since multiple negative charges on a chelator are neutralized after binding to a metal cation. Methods to perform such purifications have been described in the scientific literature.

Alternate radiometals that bind to thiol or thiol-emnino containing ligands can also be used within the scope of the invention. These radiometals include, but are not restricted to, Cu-64, Cu-67, Pd-109, Ag-111, Re-186, Re-188, Pt-197, Bi-212, Bi-213 and Pb-212.

### Haptens

Haptens carrying non-metallic therapeutic radionuclides can also be used in the method. For instance the recognition units epsilon-HSG-lysyl-tyrosine and HSG-tyrosine can be radioiodinated with the I-125 or I-131 radionuclides, and the radioiodinated recognition units can be used after msAb pretargeting. Similar agents can be prepared using radioastatine, if a therapeutic alpha-particle emitting radionuclide is desired. Newer radioiodination agents have been designed that produce a non-metabolizable form of radioiodine that is retained in cells after intracellular processing. A variety of such agents have been described in the scientific literature and they can be used to prepare conjugates of recognition haptens with residualizing radiohalogen sub-units. The preparation of conjugates of the recognition hapten and the moiety that actually carries the radionuclide uses standard techniques and methods of organic chemistry. Any appropriate chemical linkage can be used, exemplified by but not limited to, carboxyl to amino to produce an amide bond, thiol to halocarbon to produce a thioethers bond, amino to aldehyde to produce an imine bond, optionally reducible to a secondary amino bond, etc. When appropriate short linkers can be used, such as a diamine used to link a carboxyl-containing nuclide carrier (e.g. metal-DTPA) and a carboxyl-containing recognition unit (e.g. histamine-succinyl-glycine). It is understood that these general principles are applicable to all the conjugates that may be prepared for use in this invention.

Bivalent recognition haptens used in systemic msAb therapies are also useful with this intravesicular approach. Basically, any suitable chemical linkage can attach the two recognition haptens to each other. For instance, two recognition haptens linked by a short linear or cyclic peptide, as exemplified by:
Ac-Phe-Lys(DOTA)-Tyr-Lys(DOTA)-NH₂
DOTA-Phe-Lys(HSG)-Tyr-Lys(HSG)-NH₂
Ac-Phe-Lys(DTPA)-Tyr-Lys(DTPA)-NH₂
   DOTA-Phe-Lys(HSG)-D-Tyr-Lys(HSG)-NH₂
Ac-Lys(HSG)-D-Tyr-Lys(HSG)-Lys(Tscg-Cys)-NH₂
Ac-Cys-Lys(DOTA)-D-Tyr-Ala-Lys(DOTA)-Cys-NH₂

In these examples the DOTA or DTPA units can be radiolabeled with any of the same therapeutically useful radiometal radionuclides listed above that prefer oxygen-nitrogen ligands. Likewise, the chelate Tscg-Cys-(thiosemicarbazonylglyoxyl-cysteine-) is designed to be labeled with therapeutic radiometals that prefer thiol-nitrogen ligands. The peptides can be designed with tyrosyl residues already incorporated so that they can be readily iodinated with I-125 or I-131. Peptides that contain more than one carrier site that can accept a radionuclide can be double labeled, for instance with radioiodine and with a radiometal. Peptides can be chosen to be resistant to enzymes, such that they contain D-amino acids, and are N-terminal acylated and C-terminal amidated. The above species can be used with msAbs having anti-DTPA, anti-DOTA or anti-HSG secondary recognition arms, as appropriate. The same recognition units can also be readily attached to templates that are non-peptide in nature. For instance simple diamines can be doubly substituted with DTPA or DOTA moieties. An appropriately substituted diamino-sugar template can be doubly substituted with DOTA or DTPA in a similar manner.

More than two recognition units can also be use in the practice of the invention. Most preferably this is done when the recognition unit is also an integral part of the radiotherapy agent, for example, a yttrium-90-DOTA chelate complex. Such complexes can be multiply substituted onto polymeric carriers. The polymeric carriers that carry agents such as yttrium-90-DOTA and are used in this invention are preferably administered intravesically, since there is then much less concern about non-specific tissue uptake, and metabolic clearance of large amounts of radionuclide into tissues such as the liver and kidney. In a preferred embodiment, the recognition unit and the radionuclide carrier are separated such that a polymer of the type [HSG]ₘ-polymer backbone-[DOTA-yttrium-90]ₙ is generated, where HSG comprises the recognition hapten. Preferably m = 1, while n =10-100. In any event, the level of substitution of the recognition hapten is then held at 1-2 per polymer unit, while the level of the DOTA substitution is maximized per unit of polymer. This type of complex, freed from systemic pharmacokinetic concerns, can be readily super-loaded with Y-90. Since binding and recognition to tumor is via an HSG-containing msAb it can be ensured that every msAb pretargeted to the tumor will deliver at least one atom of yttrium-90 for therapeutic decay.

Any aspect of the present can be wherein the carrier molecule is a polymer of the structure [HSG]ₘ-polymer backbone-[DOTA-therapeutic agent]ₙ wherein HSG comprises a recognition hapten wherein m ≥ 1 and n ≥ 1. (M can be 1 or 2, and n can from 1 to about 100.) The method of claim 1, wherein the carrier molecule can be a biocompatible polymer. The carrier molecule can be a polyamino acid or polypeptide, wherein the amino acids are D-,L-, or both. The carrier molecule can be a polyamino acid or polypeptide selected from the group consisting of polylysine, polyglutamic acid, polyaspartic acid, a poly(Lys-Glu) co-polymer, a poly(Lys-Asp) copolymer, a poly(Lys-Ala-Glu-Tyr) (KAEY; 5:6:2:1) co-polymer or a polypeptides of from 2-50 residues chain length. The carrier molecule can be selected from the group consisting of poly(ethylene) glycol (PEG), N-(2-hydroxypropyl)methacrylamide (HMPA) copolymers, poly(styrene-co-maleic acid/anhycdride (SMA), poly(divinylether maleic anhydride) (DIVEMA), polyethyleneimine, ethoxylated polyethylemeimine, dendrimers, poly(N-vinylpyrrolidone) (PVP) epsilon-[histaminyl-succinyl-glycyl]-lysine amide, and apo-metallothionein coupled to *p*-bromoacetamido-benzyl-DTPA. The carrier molecule can be an immunogenic agent to which secondary recognition antibodies can be raised.

Conjugates and bifunctional ligands useful for the present invention include those disclosed in U.S. Patent No. 5,612,016. Also useful in the present invention are the binding ligands disclosed in U.S. Patent No. 6,126,916 and the chelating agents disclosed in U.S. Application 09/823,746, filed on April 4, 2001.

### Polymeric Carriers

Exemplary Polymeric carriers of the invention are polyamino acids (polypeptides) such as polylysine, polyglutamic (E; single letter code) and aspartic acids (D), including. D-amino acid analogs of the same. Co-polymers such as poly(Lys-Glu) {poly[KE]} are especially useful, when such co-polymers are selected with the building blocks in desirable ratios to each other. These ratios may be advantageously from 1:10 to 10:1, in the case of poly[KE] or poly[KD]. More complex co-polymers based on amino acid building blocks such as poly(Lys-Ala-Glu-Tyr) (KAEY; 5:6:2:1) may also be employed. The useful molecular weight of the polymer is generally within the range 1,000 to 100,000 Daltons. Amino acid building blocks are chosen not only for their ability to act as carriers for the recognition hapten and therapy agent, but also for the physical and biological properties that the individual building blocks can make to the overall polymer conjugates. For instance, a preferred polymer conjugate is one that retains adequate solubility even when multiply substituted. In the case of polypeptides this often means an abundance of charge residues being present Another preferred property is engendered in a final polymer conjugate that retains a net negative charge at physiological pH, since agents with net positive charges can sometimes bind non-specifically to cells and tissues. In the case of polypeptides a preponderance of acidic residues such as aspartate and glutamate most readily satisfy this criteria. A third preferred property is that the polymer backbone is stable to any enzymes that may be present in bladder tissue. For this preference, polypeptides can incorporate D-aminp acids, and will be acylated and amidated, at the N- and C-termini, respectively In terms of preferred molecular weight ranges base polymer weights between 5,000 and 25,000 are especially preferred.

Smaller polymeric carriers of completely defined molecular weight are also preferred within the scope of the invention. These can be produced as chemically defined entities by solid-phase peptide synthesis techniques, readily producing polypeptides of from 2-50 residues chain length. A second advantage of this type of reagent, other than precise structural definition, is the ability to place single or any desired number of chemical handles at certain points in the chain. These can be later used for attachment of recognition haptens and therapeutic radionuclides at chosen levels of each moiety.

Polymers other than polypeptides can be used within the scope of the invention. Poly(ethylene) glycol [PEG] has desirable *in vivo* properties for a multispecific antibody prodrug approach, and can be obtained in a variety of forms having different chemical functionalities at the ends of the polymer. Most PEG derivatives have just two functionally reactive sites, at either end of the polymer chain but branched chain units have also been made. Other synthetic polymers that can be used to carry recognition haptens and therapeutic radionuclides include N-(2-hydroxypropyl)methacrylamide (HMPA) copolymers, poly(styrene-co-maleic acid/anhydride (SMA), poly(divinylether maleic anhydride) (DIVEMA), polyethyleneimine, ethoxylated polyethyleneimine, starburst dendrimers and poly(N-vinylpyrrolidone) (PVP). As an example, DIVEMA polymer comprised of multiple anhydride units is reacted with a limited amount of amino-benzyl-DTPA to produce a desired substitution ratio of DTPA chelates on the polymer backbone. Remaining anhydride groups then are opened under aqueous conditions to produce free carboxylate groups. A limited number of the free carboxylate groups are activated using standard water-soluble peptide coupling agents (e.g. EDAC) and coupled to a recognition moiety bearing a free amino group. An example of the latter would be epsilon-[histaminyl-succinyl-glycyl]-lysine amide. (HSGK-NH₂) since antibodies have already been raised to the HSG portion of the compound. The free alpha lysine residue then becomes the point of attachment to the polymer backbone for the recognition hapten. Finally, in certain instances, the polymer used can be a naturally occurring polymer. An instance of this is the use of apo-metallothionein, which is a low MW protein having seven free thiol groups. This protein can be coupled to *p-*bromoacetamido-benzyl-DTPA to attach the DTPA units using a thioethers linkage. The protein can then have a limited number of epsilon lysyl-residues modified to carry a recognition hapten such as HSG.

The polymer backbone itself can be used as an immunogenic agent that secondary recognition mAbs can be raised against. The polymer can be attached to a macromolecule to enhance immunogenicity, and that conjugate used as an immunogen, with screening for antibody expression done using standard methods. Production of antibodies against the polymer backbone can have the advantage of producing a 'universal' recognition MAb. Thus, as when using distinct recognition units such as DTPA, HSG or DOTA, secondary antibody recognition is not tied to any particular drug, and the same msAb can be used against a variety of radiotherapy agents conjugated to the same polymer backbone. One can contemplate that this embodiment will be useful if two different polymer-radionuclide conjugates will be used in combination (in order to gain the advantage of using several nuclides of different energies in a situation that parallels combination chemotherapy. Additional polymers useful in the present invention are described in U.S. Provisional Application No. 60/308,605, filed on July 31, 2001.

### Administration

In terms of administration to a patient, the msAb pretargeting step is preferably given intravesically. The radiolabeled recognition hapten can be given either intravesically or systemically, preferably intravenously, or by a combination of both routes. The optimum time to give the radiolabeled recognition hapten is after complete or near-complete clearance of the msAb from the bladder and surrounding tissues such as the bladder wall. However, in another embodiment both agents can be given together intravesically. In this form the msAb and the radiolabeled recognition hapten are premixed prior to patient administration. An advantage of this approach is that each msAb can be ensured to bind to radiolabeled recognition hapten prior to said administration. Finally, it is understood that other agents or procedures usually given or performed to bladder emptying may also be performed to hasten clearance of any of the agents described above. Any composition administered by this invention can be a administering is via the urethra.

In any aspect of the current invention, the multispecific antibody and the conjugate can be mixed prior to administration. The multispecific antibody and conjugate can be prepared in a substantially carrier free form. The antibody and the conjugate can be mixed in approximately an equimolar ratio. Also an additional aspect is allowing any of the unbound composition to substantially clear from the patient. The the administration of the multispecific antibody can be via the urethra of the patient's bladder. The multispecific antibody can be allowed to clear from the patient's urethra by evacuation. The multispecific antibody can be cleared through a catheter. The therapeutic agent can be administered intravenously or via the urethra of the patient's bladder, or by both methods. The therapeutic agent can administered via the urethra of the patient's bladder. The therapeutic agent can be administered via the urethra of the patient's bladder at different intervals. A complex of a therapeutic agent carrier and a therapeutic agent in substantially carrier-free form can be prepared prior to administration. The multispecific antibody or therapeutic agent, or both, can be administered via the urethra. The therapeutic agent can be bound to said carrier in a substantially equimolar ratio.

The present invention can also comprises determining the amount of multispecific antibody localized into the bladder. This can be wherein the amount of multispecific antibody localized into the bladder is determined by quantifying the amount of multispecific antibody recovered from excretion. This can also be wherein the amount of multispecific antibody localized into the bladder is determined by imaging the patient and wherein the multispecific antibody further comprises a tracer nuclide.

### EXAMPLES

The example below refer to bispecific antibodies (bsAbs) which represent one type of multispecific antibody (msAb) conjugate. Examples also cite bivalent haptens as being used for delivery of the radiotherapy nuclides. The examples given are for illustrative purposes only and are not intended to be limit the scope of the present invention to only bispecific or bivalent variants of the wider class of reagents described in the specifications.

### Example 1. Preparation of a Bispecific Antibody

a) The complementary-determining region-grafted monoclonal antibody hMN-14 (humanized; anti-carcinoembryonic antigen [CEA]), and the anti-hapten antibody termed 679 (murine; anti-histaminyl-glycyl-succinimidyl- [HSG-] moiety) are separately digested to F(ab')₂ fragments by incubation for one hour with 200 ug/mL of pepsin at pH 3.7, in acetate buffer. In each case the F(ab')₂ fragment is purified from reagents and side-products by size-exclusion and ion-exchange chromatography to yield products that are substantially pure 100,000 kiloDalton fragments.
b) The F(ah')₂ fragments from the above pepsin digestions are separately incubated for one hour at 37°C in 0.1 M phosphate buffered 0.9% sodium chloride (PBS) buffer, pH 7.5, with 10 mM freshly-prepared L-cysteine. The reduced Fab'-SH fragments are separately purified by centrifugation on spin-columns containing G-50-80 SEPHADEX^{®}, equilibrated in sodium acetate buffer, pH 5.5. The product Fab' fragment antibodies are kept at 4°C prior to the cross-linking reaction.
c) The 679-Fab'-SH fragment from b) above is treated with a twenty-fold excess of the thiol-cross-linking agent *ortho*-phenyldimaleimide [OPD], dissolved in dimethyl sulfoxide, such that the final concentration of dimethyl sulfoxide in the activation reaction is 15%, and allowed to react for 30 minutes at 4°C. The product, 679-Fab'-S-linker-maleimide, is purified by centrifugation on a spin-column containing G-50-80 SEPHADEX^{®}, equilibrated in sodium acetate buffer, pH 5.5. The 679-F(ab')₂-S-linker-maleimide is mixed with a molar equivalent of the hMN-14-Fab'-SH and allowed to react at 4°C for 30 minutes. The desired product hMN-14-Fab'-linker-Fab'-679 [a Fab'₁ xFab'₂ bispecific antibody] is obtained pure by preparative size-exclusion high-performance liquid chromatography on a TSK-3000 (Tosohaas, Montgomeryville, PA), to remove low molecular weight contaminants and unreacted Fab' species.

### Example 2. Preparation of a Yttrium-90 Radiolabeled Bivalent Hapten

The mono-DOTA, di-HSG bivalent hapten peptide termed IMP 241 (DOTA-Phe-Lys(HSG)-D-Tyr-Lys(HSG)-NH₂, shown in Figure 1, is radiolabeled with Y-90 using ∼ 6 nmol of peptide and ∼ 1 mCi of dried Y-90 chloride. Six microliters of 0.25 M ammonium acetate, pH 5.4, followed by 2.7 uL (5.94 nmol) is added to a 2.2 mM solution of IMP-241 in 0.25 M ammonium acetate, pH 5.4. The solution is heated for 30-40 min at 55°C using an aluminum block heater, then quenched with 10 mM DTPA (final conc.), heated for a further 10 minutes at the same temperature, and cooled. 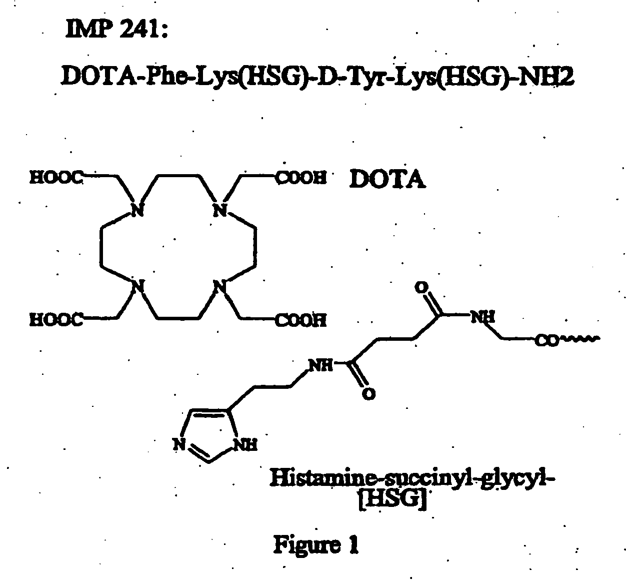 The solution is diluted with 40 uL of water, and mixed with 4.5 uL of 0.1 M aqueous triethylamine to raise the final pH to ∼ 7.5. A similar labeling is performed with In-111 acetate instead of yttrium-90 acetate.

### Example 3 Preparation of a Carrier-free Yttrium-90 Radiolabeled Bivalent Hapten

The Y-90-IMP 241 from example 2, above, is purified from non-Y-90-containing IMP 241 on Dowex AG 1-X2 anion exchange resin using gravity flow, as follows. The radiolabeled solution is paced on 0.5 nix of the resin bed in a 1-mL syringe fitted with a 2-way stopcock (the flow stopped). After 1 minute, the solution is percolated through the resin bed to just near the top of the resin bed. The flow is stopped for another minute to allow resin contact, and then continued with 10 x 0.125 mL fractions of water. Most of the applied radioactivity is recovered in fractions 4-11. Using this approach a 100-fold depletion in the level of non-Y-90-containing peptide is achieved in the final product, resulting in a specific activity of 27,888 Ci Y-90 per mmol of peptide. Since the specific activity of Y-90 itself is ∼ 500 Ci/mg (45,000 Ci/ mmol), this corresponds to 0.6 mmol of Y-90 associated with each 1 mmol of peptide, or under two molecules of peptide per molecule of Y-90 radionuclide. A second passage through AG 1-X2 resin reduces the peptide-to-yttrium-90 ratios to very close to 1:1, if desired. The Y-90-IMP 241 is then ready for injection, or is diluted further for injection or infusion.

### Example 4. Preparation of a Rhenium-188 Radiolabeled Bivalent Hapten

a) A suitable bivalent peptide is formulated for subsequent rhenium-188 labeling, as follows: The peptide IMP 192 [Ac-Lys(DTPA)-Tyr-Lys(DTPA)-Lys(Tscg-Cys)-NH₂], shown in Figure 2, is to be used for the rhenium-188 labeling.
   For formulation, 90 mL of a solution 800 mM in sodium glucoheptonate (17.85 g, 198 mg/mL) and 100 mM sodium acetate, is prepared by adding 540 mg (514 uL) of glacial acetic acid per 90 mL portion of the glucoheptonate solution. Then, 180 mg of ascorbic acid is added per 90 mL of buffer, as an anti-oxidant. To 30 mL of this mixture is added 1 mg (6.3 x 10⁻⁷ moles) of IMP-192 peptide, followed by a 6-fold molar excess of indium chloride (1.6 mL of a 2.3 x 10⁻³ molar stock solution of indium) (The indium is added to bind to the two DTPA recognition moieties, since the bispecific antibody to be used in targeting this peptide recognizes the indium-DTPA complex). To the solution is then added 90 mg of stannous chloride dihydrate, and the mixture is immediately filtered through a 0.22-micron filter, and 0.3 mL of the mixture is aliquoted into 2-mL lyophilization vials. The vials and contents, each containing 50 ug of IMP 192 peptide, are frozen using a dry ice bath, and lyophilized under vacuum. 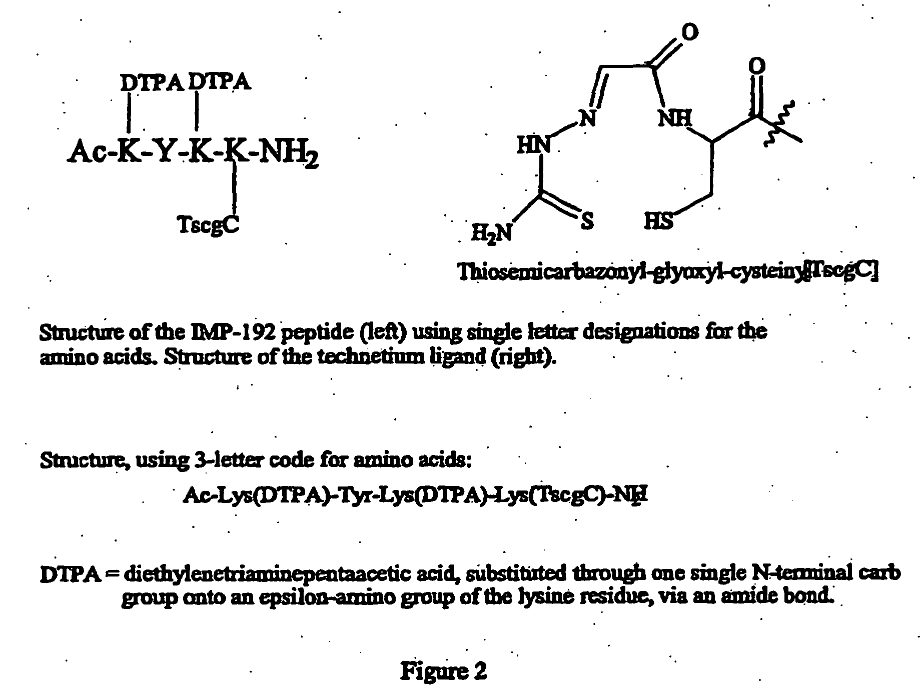
b) A concentrated Re-188 eluate (1 mL, 50 mCi), preferably taken directly from a tungsten-188/rhenium-188 radionuclide generator, is added to one of the lyophilized vials of IMP-192, part 4a) using a shielded 1-mL syringe. The vial is shaken briefly to dissolve the contents and the vial heated at 90°C for one hour. After cooling, HPLC and ITLC (instant thin-layer chromatography radioanalyses indicate a > 90% incorporation of Re-188 into the IMP 192, bound to the latter as the reduced rhenium-TscCG complex.

### Example 5. Preparation of a Carrier-free Rhenium-188 Radiolabeled Bivalent Hapten

The Re-188-IMP 192 from 4 b) above is diluted to 1:1 with 2 mL of degassed 200 mM phosphate buffered saline, pH 8.5, containing 5 mM EDTA. The diluted Re-188-IMP192 is added to the top of a SULFOLINK^{®} coupling gel column (Pierce Chemical Co., Rockford, IL), previously equilibrated with degassed 200 mM phosphate buffered saline, pH 8.5, containing 5 mM EDTA. The Re-188-IMP 192 is allowed to run onto the gel in the column, and allowed to stand in contact with the gel for 30 minutes. After this time, the buffer containing the Re-188-IMP 192 is drained from the column which is washed with a further 2 mL of degassed 200 mM phosphate buffered saline, pH 8.5, containing 5 mM EDTA. The Re-188-IMP 192 is then ready for injection, or is diluted further for injection or infusion.

### Example 6. Preparation of an Actinium-225 Radiolabeled Bivalent Hapten

The mono-DOTA, di-HSG bivalent hapten peptide termed IMP 241 (DOTA-Phe-Lys(HSG)-D-Tyr-Lys(HSG)-NH₂, shown above, is radiolabeled with Ac-225 using ∼ 6 nmol of peptide and ∼ 1 mCi of dried Ac-225. An example of a suitable salt is AcCl₃. Six microliters of 0.25 M ammonium acetate, pH 5.4, followed by 2.7 uL (5.94 nmol) is added to a 2.2 mM solution of IMP-241 in 0.25 M ammonium acetate, pH 5.4. The solution is heated for one hour at 60°C using an aluminum block heater, then quenched with 10 mM DTPA (final conc.), heated for a further 10 minutes at the same temperature, and cooled. The solution is diluted with 40 uL of water, and mixed with 4.5 uL of 0.1 M aqueous triethylamine to raise the final pH to ∼ 7.5.

### Example 7 Preparation of a Carrier-free Actinium-225 Radiolabeled Bivalent Hapten

The Ac-225-IMP 241 from example 6, above, is purified from non-actinium-225-containing IMP 241 on Dowex AG 1-X2 anion exchange resin using gravity flow, using the same procedure described in example 3), above. Using this approach a 100-fold depletion in the level of non-actinium-225-containing peptide is achieved in the final product, resulting in a peptide-to-actinium-225 ratio of under 3:1. A second passage through AG 1-X2 resin reduces the peptide-to-actinium-225 ratios to very close to 1:1, if desired. The Ac-225-IMP 241 is then ready for injection, or is diluted further for injection or infusion.

### Example 8. Preparation of a High Specific Activity Radiolabeled Polymer

a) A stirred solution of poly(L-lysine) 10 mg (about 5 x 10⁻⁸ moles; assuming an average MW of about 200,000) in 2 mL of sodium borate buffer, pH 8.5, is treated with an approximately 100-fold molar excess (∼1.8 mg) of diethylenetriaminepentaacetic acid dianhydride (DTPAA, Sigma Chem.Co., St Louis, MO). After stirring for a further 15 minutes, the pH is adjusted to 4 using dropwise addition of 2 N hydrobromic acid. After a further one hour at room temperature, the mixture is dialyzed against water in a membrane having a MW cutoff of 10,000 Daltons, to remove by-products, with four changes of dialysate being made between five 3-16 h dialyses. The solution of the product is evaporated to dryness by lyophilization to recover the title compound, which is then analyzed for amino group substitution levels by the standard TNBS (trinitrobenzensulfonic acid) assay. The product is further analyzed for DTPA chelate content by radiolabeling an accurately weighed sample with In-111/cold indium standard solution added in excess, and a determination of indium uptake versus unbound indium in the labeling mixture.
b) The DTPA-poly-(L-lysine) as prepared in 8a), above, is radiolabeled with Y-90 using at a 1:5 ratio of Y-90 to available DTPA residues, as the latter are determined from the indium binding assay. The labeling is performed in 0.25 M ammonium acetate buffer, pH 5.4, at room temperature for fifteen minutes. The labeling mixture is then treated with an equivalent of indium chloride and allowed to stand at room temperature for a further 15 minutes. The Y-90(indium-DTPA) -poly-(L-lysine) can be purified by size exclusion chromatography to remove any excess indium metal, or can be used without further purification. The Y-90-(indium-DTPA)-poly-(L-lysine) is ready for injection, or is diluted further for injection or infusion.

### Example 9. Treatment of a Bladder Cancer Patient with Premixed Bispecific Antibody-Mediated Radioimmunotherapy Using a Beta-Emitting Radionuclide

A 68-year-old male patient with a superficial cancer of the urinary bladder is treated with a 1:1 molar mixture of the bispecific antibody hMN-14 x 679-F(ab')₂ [anti-CEA x anti-HSG] of example 1, and the carrier-free Y-90-IMP 241 bivalent hapten of example 3, above. The premixed radioimmunotherapy agent is introduced into the bladder via a urethral catheter inserted under local anesthetic. Prior to injection, the bladder is drained completely, and 70 mL of the complex in 70 mL 0.9% NaCl (comprising 20 mg of the bispecific antibody and 10 mCi of Y-90 conjugated to the bivalent hapten) are instilled and allowed 90 minutes to localize by binding to tumor tissue. The unbound radiolabeled is bispecific antibody mixture is then allowed to evacuate the bladder through the urethra, by washing out the bladder using 50 mL 0.9% NaCl, leaving the remaining administered radioactivity bound substantially only to tumor cells. Seventy-two hours later, the patient is taken to the operating room, where biopsies of macroscopically normal urothelium and bladder tumor are made, The urothelium is separated from the underlying mucularis layer and assayed in a beta scintillating counter to allow measurement of radioactivity in the tumor and in the normal tissue, and then the preparations were fixed in formalin for histopathological evaluation. A count ratio of 6:1 is found for tumor:normal tissue radioactivity, and the histology specimen shows relatively intact normal urothelium but areas of marked degeration and necrosis in tumor sites, indicating onset of selective tumor lysis. Cystoscopic examination of the patient over the following three months indicates a reduction and resorption of sites of apparent cancer by more than approximately 50 percent. The patient receives a repeated administration of this therapy 6 months after the initial cone, and experiences another regression of disease by about 30 percent At one year following the initial therapy, cystoscopic examination reveals the presence of a few small foci of apparent carcinoma, but these do not seem to have grown over the time of observation and the patient appears to have minimal symptoms of bladder discomfort or evidence of blood in his urine.

### Example 10. Treatment of a Bladder Cancer Patient with Pretatargeted Bispecific Antibody-Mediated Radioimmunotherapy Using a Beta-Emitting Radionuclide

Another patient with a recurrent bladder cancer is treated with a bispecific antibody comprised of an anti-bMN-14 x anti-indium-DTPA bispecific antibody, by direct introduction of the agent into the bladder through the urethra, similar as per the prior example. During the next two hours, the patient is allowed to void regularly allowing non-antigen bound bispecific antibody to clear the organ. After two-hours to allow for specific targeting and clearance, the Re-188-IMP 192 of example 5, above, is injected, at a dose of 40 mCi, intravenously into the patient. The Re-188-radiolabeled peptide rapidly clears via the kidneys and through the bladder, binding to pretargeted bispecific antibody retained therein, while non-captured, excess Re-188-**I**MP 192 is allowed to void from the patient The patient tolerates the procedure well, and upon cystoscopic examination, with biopsies taken, 6 weeks later, evidence of reduction of size and number of cancer sites is observed, and the biopsies taken confirm selective tumor-cell necrosis.

### Example 11. Treatment of a Bladder Cancer Patient with Pretargeted Bispecific Antibody-Mediated Radioimmunotherapy Using an Alpha-Emitting Radionuclide

A patient presenting with an invasive bladder cancer, is treated with a bispecific antibody comprised of an anti-EGFR x anti-HSG bispecific antibody, by direct introduction of the agent into the bladder through the urethra, as described in example 9. After six hours, to allow for localization and urinary clearance of the bispecific antibody, the Ac-225-IMP 241 composition of example 6, above, is also introduced into the bladder via the urethra. Within one hour, all available sites of previously introduced anti-HSG antibody arms capture the introduced Ac-225-IMP 241. Any residual Ac-225-IMP 241 is allowed to void via the urethra, with optional administration of fluids to speed the clearence process.

### Example 12. Treatment of a Bladder Cancer Patient with Pretargeted Bispecific Antibody-Mediated Radioimmunotherapy Using an Alpha-Emitting Radionuclide

A patient presenting with an invasive bladder cancer, is treated with a bispecific antibody comprised of an anti-hMN-14 x anti-HSG bispecific antibody, by direct introduction of the agent into the bladder through the urethra. After six hours, to allow for localization and urinary clearance of the bispecific antibody, the Ac-225-IMP 241 composition of example 7, above, is also introduced into the bladder via the urethra. Within one hour, all available sires of previously introduced anti-HSG antibody arms capture the introduced Ac-225-IMP 241. Any residual Ac-225-IMP 241 is allowed to void via the urethra, with administration of 50 mL 0.9% NaCl to speed the clearance process.

### Example 13. Treatment of a Bladder Cancer Patient with Pretargeted Bispecific Antibody-Mediated Radioimmunotherapy Following Quantitation of Localization by Radioimmunodetection

A patient with a recurrent bladder cancer is treated with an I-131-radioiodinated bispecific antibody having of anti-MUC-1 x anti-indium-DTPA arms, by direct introduction of the agent into the bladder through the urethra. During the next two hours, the patient is allowed to void regularly allowing non-antigen bound bispecific antibody to clear the organ. After a two-hour period, to allow for specific targeting and clearance, the patient is imaged by radioimmunodetection using planar or single photon emission computed (SPECT) technique and the extent and amount of I-131 retained in diseased bladder tissue is estimated from the observed count-rate in relation to the administered dose. Re-188-IMP 192 of example 4, above, is then administered into the patient via the urethra, with the administered dose pre-calculated from the results of the prior, quantitative radioimmunoimaging. Any slight excess of Re-188-IMP 192 is allowed to clear from the patient via the normal route. The scans show specific localization of the radioisotope at the 48-hr imams, approximately in the areas of the bladder where there is known disease, and it is estimated from the scans that the tumor-to-nontumor ratios are in the range of 4:1 to 8:1.

## Claims

1. The use of a therapeutically effective amount of a bispecific antibody comprising at least one targeting arm that binds a bladder cancer antigen and at least one capture arm that binds a carrier conjugated to one or more therapeutic agents in the preparation of a medicament for the treatment of bladder cancer in a patient; wherein the bispecific antibody is administered via the urethra and is optionally allowed to localize at the site of the bladder cancer, and optionally any free bispecific antibody is allowed to substantially clear from the patient.

2. The use of claim 1, wherein the carrier-conjugated therapeutic agent is administered via the urethra.

3. The use of claim 1, further comprising determining the amount of bispecific antibody localized into the bladder prior to administering said carrier conjugated to one or more therapeutic agents.

4. The use of claim 3, wherein the amount of bispecific antibody localized into the bladder is determined by quantifying the amount of bispecific antibody recovered from excretion.

5. The use of claim 3, wherein the amount of bispecific antibody localized into the bladder is determined by imaging the patient and wherein the bispecifc antibody further comprises a tracer nuclide.

6. The use of claim 1, wherein the bispecific antibody comprises one or more antibody fragments or sub-fragments.

7. The use of claim 6, wherein the bispecific antibody is selected from the group consisting of IgG x Fab', IgG x sFv, F(ab')₂ x Fab', Fab' x Fab', Fab' x sFv, (sFv x sFv)₂, sFv x sFv, diabody, triabody, tetrabody, and quintabody.

8. The use of claim 1, wherein the bispecific antibody has more than one targeting arm.

9. The use of claim 8, wherein said more than one targeting arm is F(ab')₂ x Fab'

10. The use of claim 1, wherein said bladder cancer antigen is selected from the group consisting of carcinoembryonic antigen (CEA), CD44, MUC-1, MUC-2, MUC-3, MUC-4; Le-y, TAG-72, IL-6, epithelial glycoprotein (EGP), epidermal growth factor receptor (EGFR), vascular endothelial growth factor receptor (VEGFR), tumor necrosis substances, and human milk fat globulin antigens (HMFG1 and HMFG2).

11. The use of claim 4, wherein said tracer nuclide is selected from the group consisting of F-18, Ga-67, Ga-68, Tc-99m, In-111, I-123 and I-131, or gadolinium.

12. The use of claim 1, wherein said therapeutic agent is selecting from the group consisting of Sc-47, Ga-67, Y-90, Ag-111, In-111, Sm-153, Tb-166, Lu-177, Bi-213, Ac-225, Cu-64, Cu-67, Pd-109, Ag-111, Re-186, Re-188, Pt-197, Bi-212, Bi-213, Pb-212 and Ra-223.

13. The use of claim 1, wherein the carrier molecule is a polymer of the structure [HSG]ₘ-polymer backbone-[DOTA-therapeutic agent]ₙ wherein HSG comprises a recognition hapten wherein m ≥ 1 and n ≥1.

14. The use of claim 13, wherein m=1 or 2.

15. The use of claim 13, wherein n is from 1 to about 100.

16. The use of claim 1, wherein the carrier molecule is a biocompatible polymer.

17. The use of claim 16, wherein the carrier molecule is a polyamino acid or polypeptide, wherein the amino acids are D-, L-, or both.

18. The use of claim 17, wherein the carrier molecule is a polyamino acid or polypeptide selected from the group consisting of polylysine, polyglutamic acid, polyaspartic acid, a poly(Lys-Glu) co-polymer, a poly(Lys-Asp) copolymer, a poly(Lys-Ala-Glu-Tyr) (KAEY; 5:6:2:1) co-polymer or a polypeptide of from 2-50 residues chain length.

19. The use of claim 18, wherein the carrier molecule is selected from the group consisting of poly(ethylene) glycol (PEG), N-(2-hydroxypropyl)methacrylamide (HMPA) copolymers, poly(styrene-co-maleic acid/anhydride (SMA), poly(divinylether maleic anhydride) (DIVEMA), polyethyleneimine, ethoxylated polyethyleneimine, dendrimers, poly(N-vinylpyrrolidone) (PVP) epsilon-[histaminyl-succinyl-glycyl]-lysine amide, and apo-metallothionein coupled to *p-*bromoacetamido-benzyl-DTPA.

20. The use of claim 19, wherein the carrier molecule is an immunogenic agent to which secondary recognition antibodies can be raised.

21. The use of claim 1, wherein the bispecific antibody and the conjugate are mixed prior to administration.

22. The use of claim 21, wherein the bispecific antibody and conjugate are prepared in a substantially carrier free form.

23. The use of claim 22, wherein the antibody and the conjugate are mixed in approximately an equimolar ratio.

24. The use of claim 1, wherein the bispecific antibody is allowed to clear from the patient's urethra by evacuation.

25. The use of claim 24, wherein the bispecific antibody is cleared through a catheter.

26. The use of claims I or 21, wherein the therapeutic agent is administered intravenously or via the urethra of the patient's bladder, or by both methods.

27. The use of claim 21, wherein the therapeutic agent is administered via the urethra of the patient's bladder.

28. The use of a composition comprising a therapeutically effective amount of a bispecific antibody comprising at least one targeting arm that binds a bladder cancer antigen and at least one capture arm that binds a carrier conjugated to at least one or more therapeutic agent/s in the preparation of a medicament for the treatment of bladder cancer in a patient; wherein the bispecific antibody is administered via the urethra.

29. The use of claim 28, wherein the bispecific antibody is a fragment or subfragment.

30. The use of claim 29, wherein the bispecific antibody is a fragment or subfragment is selected from the group consisting of IgG x Fab', IgG x sFv, F(ab')_{2.}x Fab', Fab' x Fab', Fab' x sFv, (sFv x sFv)₂, sFv x sFv, diabody, triabody, tetrabody and quintabody.

31. The use of claim 30, wherein the bispecific antibody has more than one targeting arm.

32. The use of claim 31, wherein said more than one targeting arm is F(ab')₂ x Fab'.

33. The use of claim 32, wherein said bladder cancer antigen is selected from the group consisting of carcinoembyronic (CEA), CD44, MUC-1, MUC-2, MUC-3, MUC-4; Le-y, TAG-72, IL-6, epithelial glycoprotein (EGP), epidermal growth factor receptor (EGFR), vascular endothelial growth factor receptor (VEGFR), tumor necrosis substances, and human milk fat globulin antigens (HMFG1 and MHFG2).

34. The use of claim 33, wherein the carrier molecule is an immunogenic agent to which secondary recognition antibodies can be raised.

35. The use of claim 1 wherein the therapeutic agent is a toxin, a chemotherapeutic drug, or a chemotherapeutic drug conjugated to one or more haptens.

## Patentansprüche

1. Verwendung einer therapeutisch wirksamen Menge eines bispezifischen Antikörpers, der wenigstens einen targetierenden Arm, welcher ein Blasenkrebsantigen bindet, und wenigstens einen Einfangarm umfasst, der einen mit einem oder mehr als einem therapeutischen Agens konjugierten Träger bindet, bei der Herstellung eines Medikaments zur Behandlung von Blasenkrebs bei einem Patienten; wobei der bispezifische Antikörper über die Harnröhre verabreicht und ihm optional erlaubt wird, sich an der Stelle des Blasenkrebses zu lokalisieren, und man optional erlaubt, dass ein jeglicher freier bispezifischer Antikörper im Wesentlichen aus dem Patienten entfernt wird.

2. Verwendung nach Anspruch 1, wobei das mit dem Träger konjugierte therapeutische Agens über die Harnröhre verabreicht wird.

3. Verwendung nach Anspruch 1, weiter umfassend das Bestimmen der Menge von in der Blase lokalisiertem bispezifischen Antikörper vor dem Verabreichen des mit einem oder mehr als einem therapeutischen Agens konjugierten Trägers.

4. Verwendung nach Anspruch 3, wobei die Menge von in der Blase lokalisiertem bispezifischen Antikörper durch das Quantifizieren der Menge an aus der Ausscheidung gewonnenem bispezifischen Antikörper bestimmt wird.

5. Verwendung nach Anspruch 3, wobei die Menge von in der Blase lokalisiertem bispezifischen Antikörper bestimmt wird, indem der Patient einem bildgebenden Verfahren unterzogen wird und wobei der bispezifische Antikörper weiterhin ein Tracernuklid umfasst.

6. Verwendung nach Anspruch 1, wobei der bispezifische Antikörper ein oder mehr als ein Antikörperfragment oder Subfragment umfasst.

7. Verwendung nach Anspruch 6, wobei der bispezifische Antikörper aus der Gruppe ausgewählt ist, die aus IgG x Fab', IgG x sFv, F(ab')₂ x Fab', Fab' x Fab', Fab' x sFv, (sFv x sFv)₂, sFv x sFv, einem Diabody, Triabody, Tetrabody und Quintabody besteht.

8. Verwendung nach Anspruch 1, wobei der bispezifische Antikörper mehr als einen targetierenden Arm aufweist.

9. Verwendung nach Anspruch 8, wobei der mehr als eine targetierende Arm F(ab')₂ x Fab' ist.

10. Verwendung nach Anspruch 1, wobei das Blasenkrebsantigen aus der Gruppe ausgewählt ist, die aus karzinoembryonalem Antigen (CEA), CD44, MUC-1, MUC-2, MUC-3, MUC-4, Le-y, TAG-72, IL-6, epithelialem Glykoprotein (EGP), epidermalem Wachstumsfaktor-Rezeptor (EGFR), vaskulärem endothelialem Wachstumsfaktor-Rezeptor (VEGFR), Tumomekrosesubstanzen und menschlichen Milchfett-Globulin-Antigenen (HMFG1 und HMFG2) besteht.

11. Verwendung nach Anspruch 4, wobei das Tracernuklid aus der Gruppe, die aus F-18, Ga-67, Ga-68, Tc-99m, In-111, I-123 und I-131 besteht, ausgewählt ist oder Gadolinium ist.

12. Verwendung nach Anspruch 1, wobei das therapeutische Agens aus der Gruppe auswählt, die aus Sc-47, Ga-67, Y-90, Ag-111, In-111, Sm-153, Tb-166, Lu-177, Bi-213, Ac-225, Cu-64, Cu-67, Pd-109, Ag-111, Re-186, Re-188, Pt-197, Bi-212, Bi-213, Pb-212 und Ra-223 besteht.

13. Verwendung nach Anspruch 1, wobei das Trägermolekül ein Polymer der Struktur [HSG]ₘ-Polymer-Rückgrat-[DOTA-therapeutisches Agens]ₙ ist, wobei HSG ein Erkennungshapten umfasst, wobei m ≥1 und n ≥1.

14. Verwendung nach Anspruch 13, wobei m = 1 oder 2.

15. Verwendung nach Anspruch 13, wobei n von 1 bis etwa 100 ist.

16. Verwendung nach Anspruch 1, wobei das Trägermolekül ein biokompatibles Polymer ist.

17. Verwendung nach Anspruch 16, wobei das Trägermolekül eine Polyaminosäure oder ein Polypeptid ist, wobei die Aminosäuren D-, L-Aminosäuren oder sowohl D- als auch L-Aminosäuren sind.

18. Verwendung nach Anspruch 17, wobei das Trägermolekül eine Polyaminosäure oder ein Polypeptid ist, die/das aus der Gruppe ausgewählt ist, die aus Polylysin, Polyglutaminsäure, Polyasparaginsäure, einem Poly (Lys-Glu)-Co-Polymer, einem Poly (Lys-Asp)-Co-Polymer, einem Poly (Lys-Ala-Glu-Tyr) (KAEY; 5:6:2:1)-Co-Polymer oder einem Polypeptid mit einer Kettenlänge von 2-50 Resten besteht.

19. Verwendung nach Anspruch 18, wobei das Trägermolekül aus der Gruppe ausgewählt ist, die aus Poly(ethylen)glykol (PEG), N-(2-Hydroxypropyl)methacrylamid (HMPA)-Copolymeren, Poly(styrol-co-maleinsäure/anhydrid (SMA), Poly(divinylethermaleinanhydrid) (DIVEMA), Polyethylenimin, ethoxyliertem Polyethylenimin, Dendrimeren, Poly (N-vinylpyrrolidon) (PVP) epsilon-[histaminyl-succinyl-glcyl]-Lysinamid und an *p*-Bromacetamido-benzyl-DTPA gekoppeltes Apo-Metallothionein besteht.

20. Verwendung nach Anspruch 19, wobei das Trägermolekül ein immunogenes Agens ist, gegen das sekundäre Erkennungsantikörper erzeugt werden können.

21. Verwendung nach Anspruch 1, wobei der bispezische Antikörper und das Konjugat vor der Verabreichung vermischt werden.

22. Verwendung nach Anspruch 21, wobei der bispezifische Antikörper und das Konjugat in einer im Wesentlichen trägerfreien Form hergestellt werden.

23. Verwendung nach Anspruch 22, wobei der Antikörper und das Konjugat in einem ungefähr äquimolaren Verhältnis vermischt sind.

24. Verwendung nach Anspruch 1, wobei man dem bispezifischen Antikörper erlaubt, sich durch Entleerung aus der Harnröhre des Patienten zu entfernen.

25. Verwendung nach Anspruch 24, wobei der bispezifische Antikörper durch einen Katheter entfernt wird.

26. Verwendung nach den Ansprüchen 1 oder 21, wobei das therapeutische Agens intravenös oder über die Hamöhre der Blase des Patienten oder durch beide Verfahren verabreicht wird.

27. Verwendung nach Anspruch 21, wobei das therapeutische Agens über die Harnröhre der Blase des Patienten verabreicht wird.

28. Verwendung einer Zusammensetzung, die eine therapeutisch wirksame Menge eines bispezifischen Antikörpers umfasst, der wenigstens einen targetierenden Arm, welcher ein Blasenkrebsantigen bindet, und wenigstens einen Einfangarm umfasst, welcher einen mit einem oder mehr als einem therapeutischen Agens konjugierten Träger bindet, bei der Herstellung eines Medikaments zur Behandlung von Blasenkrebs bei einem Patient; wobei der bispezifische Antikörper über die Harnröhre verabreicht wird.

29. Verwendung nach Anspruch 28, wobei der bispezifische Antikörper ein Fragment oder Subfragment ist.

30. Verwendung nach Anspruch 29, wobei der bispezifische Antikörper ein Fragment oder ein Subfragment ist, das aus der Gruppe ausgewählt ist, die aus IgG x Fab', IgG x sFv, F(ab')₂ x Fab', Fab' x Fab', Fab' x sFv, (sFv x sFv)₂, sFv x sFv, einem Diabody, Triabody, Tetrabody und Quintabody besteht.

31. Verwendung nach Anspruch 30, wobei der bispezifische Antikörper mehr als einen targetierenden Arm aufweist.

32. Verwendung nach Anspruch 31, wobei der mehr als eine targetierende Arm F(ab')₂ x Fab' ist.

33. Verwendung nach Anspruch 32, wobei das Blasenkrebsantigen aus der Gruppe ausgewählt ist, die aus karzinoembryonalem Antigen (CEA), CD44, MUC-1, MUC-2, MUC-3, MUC-4; Le-y, TAG-72, IL-6, epithelialem Glykoprotein (EGP), epidermalem Wachstumsfaktor-Rezeptor (EGFR), vaskulärem endothelialem Wachstumsfaktor-Rezeptor (VEGFR), Tumornekrosesubstanzen und menschlichen Milchfett-Globulin-Antigenen (HMFG1 und HMFG2) besteht.

34. Verwendung nach Anspruch 33, wobei das Trägermolekül ein immunogenes Agens ist, gegen das sekundäre Erkennungsantikörper erzeugt werden können.

35. Verwendung nach Anspruch 1, wobei das therapeutische Agens ein Toxin, ein chemotherapeutischer Wirkstoff, oder ein an ein oder mehr als ein Hapten konjugierter chemotherapeutischer Wirkstoff ist.

## Revendications

1. Utilisation d'une quantité thérapeutiquement efficace d'un anticorps bispécifique comprenant au moins un bras de ciblage qui se lie à un antigène de cancer de la vessie et au moins un bras de capture qui se lie à un support conjugué à un ou plusieurs agents thérapeutiques dans la préparation d'un médicament pour le traitement du cancer de la vessie chez un patient ; l'anticorps bispécifique étant administré par l'intermédiaire de l'urètre et pouvant éventuellement se localiser sur le site du cancer de la vessie, et éventuellement tout anticorps bispécifique libre pouvant être essentiellement évacué par le patient.

2. Utilisation selon la revendication 1, dans laquelle l'agent thérapeutique conjugué au support est administré par l'intermédiaire de l'urètre.

3. Utilisation selon la revendication 1, comprenant en outre la détermination de la quantité d'anticorps bispécifique localisée dans la vessie avant d'administrer ledit support conjugué à un ou plusieurs agents thérapeutiques.

4. Utilisation selon la revendication 3, dans laquelle la quantité d'anticorps bispécifique localisée dans la vessie est déterminée par la quantification de la quantité d'anticorps bispécifique récupérée à partir de l'excrétion.

5. Utilisation selon la revendication 3, dans laquelle la quantité d'anticorps bispécifique localisée dans la vessie est déterminée par l'imagerie du patient et dans laquelle l'anticorps bispécifique comprend en outre un nucléide marqueur.

6. Utilisation selon la revendication 1, dans laquelle l'anticorps bispécifique comprend un ou plusieurs fragments ou sous-fragments d'anticorps.

7. Utilisation selon la revendication 6, dans laquelle l'anticorps bispécifique est choisi dans le groupe constitué par IgG x Fab', IgG x sFv, F(ab')₂ x Fab', Fab' x Fab', Fab' x sFv, (sFv x sFv)₂, sFv x sFv, un dianticorps, un trianticorps, un tétraanticorps et un pintaanticorps.

8. Utilisation selon la revendication 1, dans laquelle l'anticorps bispécifique possède plusieurs bras de ciblage.

9. Utilisation selon la revendication 8, dans laquelle lesdits plusieurs bras de ciblage sont F(ab')₂ x Fab'.

10. Utilisation selon la revendication 1, dans laquelle ledit antigène de cancer de la vessie est choisi dans le groupe constitué par un antigène carcino-embryonnaire (CEA), CD44, MUC-1, MUC-2, MUC-3, MUC-4; Le-y, TAG-72, IL-6, une glycoprotéine épithéliale (EGP), un récepteur de facteur de croissance de l'épiderme (EGFR), un récepteur de facteur de croissance endothélial vasculaire (VEGFR), des substances de nécrose de tumeur et des antigènes de globules de matière grasse du lait humain (HMFG1 et HMFG2).

11. Utilisation selon la revendication 4, dans laquelle ledit nucléide marqueur est choisi dans le groupe constitué par F-18, Ga-67, Ga-68, Tc-99m, In-111, I-123 et I-131 ou le gadolinium.

12. Utilisation selon la revendication 1, dans laquelle ledit agent thérapeutique est choisi dans le groupe constitué par Sc-47, Ga-67, Y-90, Ag-111, In-111, Sm-153, Tb-166, Lu-177, Bi-213, Ac-225, Cu-64, Cu-67, Pd-109, Ag-111, Re-186, Re-188, Pt-197, Bi-212, Bi-213, Pb-212 et Ra-223.

13. Utilisation selon la revendication 1, dans laquelle la molécule de support est un polymère répondant à la structure [HSG]ₘ-squelette de polymère-[DOTA-agent thérapeutique]ₙ dans laquelle HSG comprend un haptène de reconnaissance et dans laquelle m ≥ 1 et n ≥ 1.

14. Utilisation selon la revendication 13, dans laquelle m = 1 ou 2.

15. Utilisation selon la revendication 13, dans laquelle n vaut de 1 à environ 100.

16. Utilisation selon la revendication 1, dans laquelle la molécule de support est un polymère biocompatible.

17. Utilisation selon la revendication 16, dans laquelle la molécule de support est un poly(acide aminé) ou un polypeptide, où les acides aminés sont D-, L- ou les deux.

18. Utilisation selon la revendication 17, dans laquelle la molécule de support est un poly(acide aminé) ou un polypeptide choisi dans le groupe constitué par une polylysine, un poly(acide glutamique), un poly(acide aspartique), un copolymère de type poly(Lys-Glu), un copolymère de type poly(Lys-Asp), un copolymère de type poly(Lys-Ala-Glu-Tyr) (KAEY; 5/6/2/1) ou un polypeptide de longueur de chaîne de 2 à 50 résidus.

19. Utilisation selon la revendication 18, dans laquelle la molécule de support est choisie dans le groupe constitué par le polyéthylène glycol (PEG), des copolymères de N-(2-hydroxypropyl)méthacrylamide (HMPA), un copolymère de styrène et d'acide/anhydride maléique (SMA), un copolymère d'éther divinylique et d'anhydride maléique (DIVEMA), un polyéthylèneimine, un polyéthylèneimine éthoxylé, des dendrimères, une poly(N-vinylpyrrolidone) (PVP) epsilon-[histaminyl-succinyl-glycyl]-lysine amide et l'apo-métallothionéine couplée au bromoacétamido-benzyl-DTPA.

20. Utilisation selon la revendication 19, dans laquelle la molécule de support est un agent immunogène à partir duquel peut se développer des anticorps de reconnaissance secondaire.

21. Utilisation selon la revendication 1, dans laquelle l'anticorps bispécifique et le conjugué sont mélangés avant l'administration.

22. Utilisation selon la revendication 21, dans laquelle l'anticorps bispécifique et le conjugué sont préparés sous une forme sensiblement sans support.

23. Utilisation selon la revendication 22, dans laquelle l'anticorps et le conjugué sont mélangés dans un rapport approximativement équimolaire.

24. Utilisation selon la revendication 1, dans laquelle l'anticorps bispécifique peut être éliminé par l'intermédiaire de l'urètre du patient par évacuation.

25. Utilisation selon la revendication 24, dans laquelle l'anticorps bispécifique est éliminé à travers un cathéter.

26. Utilisation selon la revendication 1 ou 21, où l'agent thérapeutique est administré par voie intraveineuse ou par l'intermédiaire de l'urètre de la vessie du patient, ou par les deux approches.

27. Utilisation selon la revendication 21, dans laquelle l'agent thérapeutique est administré par l'intermédiaire de l'urètre de la vessie du patient.

28. Utilisation d'une composition comprenant une quantité thérapeutiquement efficace d'un anticorps bispécifique comprenant au moins un bras de ciblage qui se lie à un antigène de cancer de la vessie et au moins un bras de capture qui se lie à un support conjugué à un ou plusieurs agents thérapeutiques dans la préparation d'un médicament pour le traitement du cancer de la vessie chez un patient ; l'anticorps bispécifique étant administré par l'intermédiaire de l'urètre.

29. Utilisation selon la revendication 28, dans laquelle l'anticorps bispécifique est un fragment ou sous-fragment.

30. Utilisation selon la revendication 29, dans laquelle l'anticorps bispécifique est un fragment ou sous-fragment qui est choisi dans le groupe constitué par IgG x Fab', IgG x sFv, F(ab')₂ x Fab', Fab' x Fab', Fab' x sFv, (sFv x sFv)₂, sFv x sFv, un dianticorps, un trianticorps, un tétraanticorps et un pintaanticorps.

31. Utilisation selon la revendication 30, dans laquelle l'anticorps bispécifique possède plusieurs bras de ciblage.

32. Utilisation selon la revendication 31, dans laquelle lesdits plusieurs bras de ciblage sont F(ab')₂ x Fab'.

33. Utilisation selon la revendication 32, dans laquelle ledit antigène de cancer de la vessie est choisi dans le groupe constitué par un antigène carcino-embryonnaire (CEA), CD44, MUC-1, MUC-2, MUC-3, MUC-4; Le-y, TAG-72, IL-6, une glycoprotéine épithéliale (EGP), un récepteur de facteur de croissance de l'épiderme (EGFR), un récepteur de facteur de croissance endothélial vasculaire (VEGFR), des substances de nécrose de tumeur et des antigènes de globules de matière grasse du lait humain (HMFG1 et HMFG2).

34. Utilisation selon la revendication 33, dans laquelle la molécule de support est un agent immunogène à partir duquel peut se développer des anticorps de reconnaissance secondaire.

35. Utilisation selon la revendication 1, dans laquelle l'agent thérapeutique est une toxine, un médicament chimiothérapeutique ou un médicament chimiothérapeutique conjugué à un ou plusieurs haptènes.
